# EUROPÄISCHE PATENTSCHRIFT

(11) **EP 0 746 269 B1**
(45) Veröffentlichungstag und Bekanntmachung des Hinweises auf die Patenterteilung: **29.03.2000**
(21) Anmeldenummer: 95942039.9
(22) Anmeldetag: 19.12.1995
(51) Int. Cl.: A61F 2/06, A61L 31/00

(54) **PLATZHALTER ZUM ANORDNEN IN EINER KÖRPERRÖHRE**
STENT TO BE SET INSIDE A BODY VESSEL
EXTENSEUR DESTINE A ETRE PLACE DANS UN VAISSEAU DU CORPS

(30) Priorität: 23.12.1994 DE 4446034; 06.07.1995 DE 19524653
(43) Veröffentlichungstag der Anmeldung: 11.12.1996
(73) Patentinhaber: WILLY RÜSCH AG, D-71394 Kernen (DE)
(72) Erfinder: FREITAG, Lutz, D-58675 Hemer (DE)
(74) Vertreter: Bockermann, Rolf, Dipl.-Ing.
(86) Internationale Anmeldenummer: DE9501848
(87) Internationale Veröffentlichungsnummer: WO9619953

(56) Entgegenhaltungen:
- EP-A- 0 587 197
- US-A- 5 147 385

## Beschreibung

Die Erfindung betrifft einen Platzhalter (Stent) zum Anordnen in einer Körperröhre mit einem flexiblen Stützgerüst.

Platzhalter werden bei der Behandlung von Stenosen eingesetzt. Eine Stenose ist eine angeborene oder erworbene Verengung in einer Körperröhre. Sie kann Hohlorgane, wie beispielsweise Luftröhre, Speiseröhre oder auch Blutgefäße betreffen und als Folge von Krankheitsprozessen hervorgerufen werden. Bei entsprechender Ausprägung bewirkt eine Stenose in der betroffenen Körperröhre eine Stauung oder sogar den Verschluß derselben.

Es ist bekannt, zum Aufweiten bzw. Offenhalten von Stenosen, sei es in Blutgefäßen oder in anderen Hohlorganen, sogenannte Stents zu legen. Diese werden in der Regel mittels eines Katheters implantiert. Nach der Implantation werden sie aus dem für das Einführen erforderlichen kleinen Lumen auf ein der Gebrauchslage entsprechendes größeres Lumen verändert. Die Stents halten dann als innere Stütze das Gefäß offen und fungieren damit als Platzhalter.

Platzhalter (Stents) zum Offenhalten von Stenosen sind in vielfältigen Ausführungsformen bekannt. Es gibt sie aus Metall und/oder Kunststoffmaterial. Meist bestehen sie aus einem Geflecht von Metalldrähten mit Selbstexpansionseigenschaften.

Selbstaufdehnende Platzhalter sind beispielsweise in der EP-A-0 183 372 und in der US-PS 4 732 152 vorbeschrieben. Diese Platzhalter werden vor der Implantation gegen ihre eigenen Federrückstellkräfte auf einen reduzierten Querschnitt zusammengedrückt und mittels eines Katheters in den Körper eines Patienten eingeführt. Nach der Plazierung in der Körperröhre federn die Platzhalter durch ihre Eigenspannung auf und werden dadurch fixiert.

In der EP-A-0 177 330 ist eine Vorrichtung beschrieben, mit der ein zum Zwecke der Implantation auf einen reduzierten Querschnitt zusammengedrückter Platzhalter mit Hilfe eines Katheters in eine Körperröhre eingebracht wird und dann aus dem Katheter herausgeschoben wird.

Es gibt aber auch solche Platzhalter, die erst an ihrem Einsatzort mittels einer geeigneten Vorrichtung, beispielsweise einem Ballonkatheter, in ihre aufgeweitete Stellung expandiert werden müssen. Die US-A-5 147 395 offenbart einen Stent aus einem synthetischen Material, welches in einem Temperaturbereich zwischen 45 °C und 75 °C plastifiziert und verformbar wird. Der Stent wird an seine vorgesehene Stelle in einer Körperröhre gebracht und mittels eines heizbaren Ballonkatheters erhitzt, bis er in den plastifizierten Zustand übergeht. In diesem Zustand wird der Stent durch Dilatation des Ballons aufgeweitet. Nach Abkühlung auf Körpertemperatur behält der Stent die ihm gegebene Form.

Weiterhin sind auch Platzhalter bekannt (EP 0 587 197 oder DE-GM 91 16 881), bei denen die stützenden Metallsegmente in einem geschlossenen Mantel aus gewebekompatiblen Grundstoffen, beispielsweise Silikon, eingebettet sind, um so ein Durchwachsen der Platzhalter durch Gewebezellen zu verhindern.

Bekannt sind auch Platzhalter mit Metalldrähten, die aus einer sogenannten Formgedächtnislegierung (Memory-Metall) bestehen. Diese Platzhalter haben bei einer tiefen Temperatur einen geringen radialen Durchmesser. In diesem Zustand werden sie in der Stenose plaziert. Sie weiten sich dann bei Überschreiten einer Grenztemperatur, welche unter Körpertemperatur liegt, radial auf, so daß sie auf diese Weise eine Stenose offenhalten können.

Die vorgenannten Ausführungsformen verbindet alle der Nachteil, daß ihr Einführvorgang aufwendig ist. Einmal kann sich der Platzhalter durch seine Flexibilität beim Einführen verbiegen und festsetzen, andererseits sind für das Einführen auch teilweise komplizierte Einführinstrumente erforderlich, die den Platzhalter von außen umgreifen und ihn am Ort seiner Plazierung freigeben müssen.

Darüberhinaus wird es als nachteilig empfunden, daß sich die Platzhalter beim Zusammendrücken verlängern und entsprechend wieder verkürzen, wenn der Druck fortgenommen wird. Dies führt zu Problemen bei der Planung der Behandlung einer Stenose, weil hier die Länge des Platzhalters exakt abgestimmt werden muß. Es kann aber auch in der Körperröhre zu Relativbewegungen zwischen dem Platzhalter und der Gefäßwandung kommen.

Der Erfindung liegt ausgehend vom Stand der Technik die Aufgabe zugrunde, einen Platzhalter hinsichtlich seiner Ausgestaltung sowie seiner Eigenschaften zu verbessern, wobei sich dieser Platzhalter besser, einfacher und schonender im Körper plazieren läßt.

Eine vorteilhafte Lösung dieser Aufgabe besteht nach der Erfindung in dem im kennzeichnenden Teil des Anspruchs 1 aufgeführten Merkmal.

Kernpunkt ist die Verwendung eines Elastomers zur Herstellung des Stützgerüstes, welches bei Raumtemperatur hart, jedoch bei Körpertemperatur weich ist. Ein solches temperaturempfindliches Elastomer weist einmal die für die Platzierung des Platzhalters erforderliche Steifigkeit auf, andererseits erhält der Platzhalter eine gewebefunktionale Beweglichkeit, nachdem er in die Körperröhre eingesetzt worden ist.

Das Elastomer muß bei einer Temperatur von 22 °C in jedem Fall hart sein. Der Übergangsbereich vom harten zum weichen Zustand findet dann in einem Temperaturbereich von 25 °C bis 35 °C statt. Aus medizinischer und chirurgischer Sicht erweist sich ein Elastomer, bei dem der Übergang vom quasi harten zum quasi weichen Zustand bei einer Temperatur von 33 °C bis 34 °C stattfindet, als besonders vorteilhaft.

Temperaturempfindliche Elastomere stehen als solche zur Verfügung. Durch die DE-A-4 243 799 ist die Verwendung für einen Katheter bekannt.

Ein Stent kann aus einem oder mehreren Fäden in einem Flecht-, Web- oder Strickprozeß hergestellt sein. Die Fäden bestehen aus einem temperaturempfindlichen Polyurethan mit den zuvor beschriebenen Eigenschaften.

Im Stützgerüst sind zusätzlich Fäden bzw. Stränge aus einem Memory-Metall angeordnet. Vorzugsweise handelt es sich hierbei um eine Nickel-Titan-Legierung, das sogenannte Nitinol. Dieses Material hat bei einer tiefen Temperatur eine komprimierte Struktur. Es dehnt sich jedoch bei Überschreiten einer Grenztemperatur aus. Die jeweils gewünschten Grenzbedingungen können durch die entsprechende Wahl der Legierungskomponenten eingestellt werden.

Die Fäden aus dem Memory-Metall bzw. Nitinol sind bei Raumtemperatur weich, die Fäden aus dem temperaturempfindlichen Elastomer dagegen hart. Durch die Kombination ergibt sich ein Platzhalter, welcher bei Raumtemperatur dünn und hart ist. Bei Körpertemperatur wird das Elastomer weich und flexibel, während sich das Nitinol entfaltet. Es entsteht dann ein an das Lumen der Körperröhre angepaßter harter, aber elastischer Platzhalter.

Der Platzhalter kann in seinem dünnen und harten Zustand problemlos in eine Körperröhre eingeführt und dort plaziert werden. Ein Einführinstrument ist für diesen Platzhalter nicht erforderlich.

Ein solcher Stent bringt zusätzlich im wesentlichen drei weitere Vorteile mit sich. Einmal ist ein Feuchtigkeitsdurchgang durch das Stützgerüst gewährleistet. Dies bewirkt, daß im Stent, also innerhalb des Lumens ein Feuchtigkeitsfilm aufgebaut wird. Der Aufbau des Feuchtigkeitsfilms erfolgt durch den Feuchtigkeitsübertritt aus der äußeren Schleimhaut infolge einer Art passiver Diffusion. An der inneren Wandung des Stents ist so stets ein Schmierfilm vorhanden.

Desweiteren kann die Maschengröße den jeweiligen Anforderungen entsprechend variiert werden. Besonders vorteilhaft ist hierbei, daß die Maschengröße so eingestellt werden kann, daß eine gewünschte Gewebebildung ermöglicht wird. Bei einem Bronchialsystem beispielsweise ist damit das Durchwachsen mit funktionsfähigem Flimmerepithel gemeint (Epithealisieren). Bösartige Gewebezellen dagegen werden zurückgehalten.

Der weitere Vorteil betrifft das Biege- und Formverhalten des Stents. Durch die hohlzylindrische eigenstabile geflochtene Ausbildung ist das Stützgerüst bzw. der Platzhalter so flexibel, daß er gut um Krümmungen im Gefäß bzw. dem Körperlumen geführt werden kann. Der Platzhalter zeichnet sich demzufolge durch sein knickstabiles Verhalten aus.

Grundsätzlich ist es möglich, das Stützgerüst so eng zu flechten, daß das Maschengitter nicht von Gewebezellen durchwachsen werden kann. Infolge dessen wird ein erneuter teilweiser oder vollständiger Verschluß des Gefäßes vermieden.

Die zweite Lösung der erfindungsgemäßen Aufgabe besteht in den kennzeichnenden Merkmalen des Anspruchs 2.

Hier ist ein flexibles Stützgerüst in einen hohlzylindrischen Mantel eingebettet. Der Mantel besteht aus einem Memory-Elastomer mit den weiter oben beschriebenen temperaturabhängigen Eigenschaften. Dieses Elastomer ist bei Raumtemperatur hart und bei Körpertemperatur weich.

Kernpunkt ist hier die Verwendung eines Memory-Elastomers für den Mantel eines Platzhalters. Hierbei handelt es sich um ein temperaturabhängiges Elastomer, insbesondere auf Polyurethanbasis, welches eine Formgedächtniseigenschaft besitzt. Im kalten Zustand ist dieses Elastomer hart und klein. Ein daraus gebildeter Platzhalter kann dann problemlos eingeführt werden. Erst bei Körpertemperatur bzw. geringfügig unterhalb kommt es zu einer Expansion des Memory-Elastomers und mithin des Platzhalters.

Nach den Merkmalen des Anspruchs 3 wird das Stützgerüst von metallischen Drähten gebildet. Das Stützgerüst kann beliebig aufgebaut und konfiguriert sein. Es kann netzartig gewebt sein oder auch aus einzelnen Drähten bestehen, welche ohne Kontakt zueinander sind oder untereinander verknüpft sind.

Das Stützgerüst muß lediglich die Anforderung erfüllen, daß es den Expansionsvorgang des Mantels aus Memory-Elastomer zuläßt. Diese Forderung läßt sich aber durch die geeignete Ausbildung des Stützgerüstes problemlos erfüllen.

Eine den allgemeinen Erfindungsgedanken weiterbildende vorteilhafte Ausführungsform der Erfindung ist in Anspruch 4 charakterisiert. Danach bestehen die Drähte des Stützgerüstes aus einem Memory-Metall. Auch hier kommt bevorzugt Nitinol zur Anwendung. Bei der vorliegenden Ausführungsform werden Drähte aus Nitinol in einen Mantel aus Memory-Elastomer eingebettet. Die Drähte aus Nitinol sind bei Raumtemperatur weich. Der Mantel ist dagegen hart. Durch die Kombination ergibt sich wiederum ein Platzhalter, welcher bei Raumtemperatur dünn und hart ist. Bei Körpertemperatur wird das Memory-Elastomer weich und flexibel, während sich das Stützgerüst aus Nitinol entfaltet. Es entsteht dann ein dicker, harter, aber elastischer Platzhalter.

Der Einführvorgang und die Plazierung im dünnen und harten Zustand des Platzhalters ist einfach und präzise ohne ein spezielles Einführinstrument durchzuführen. Besonders vorteilhaft wirkt sich beim Einführvorgang aus, daß Nitinol im kalten Zustand verformbar ist, das heißt plastisch weiche Eigenschaften aufweist.

Die Herstellung des Platzhalters erfolgt in der Weise, daß das Memory-Metall in einem Zustand, in dem es noch weich und formbar ist, mit dem Memory-Elastomer zusammengebracht wird, wenn dieses schon weich ist. Demzufolge wird das Stützgerüst in seinem engen kleinen Zustand in das Elastomer eingegossen. Wichtig ist, daß der Herstellprozeß in einem Temperaturbereich durchgeführt wird, bei dem die beiden zusammenzufügenden Materialien ihren Körperzustand noch nicht gewechselt haben.

Auf eine vorteilhafte Ausgestaltung des Stützgerüstes in Verbindung mit dem Mantel aus Memory-Elastomer richten sich die Ansprüche 5 bis 8.

Gemäß den Merkmalen des Anspruchs 5 wird das Stützgerüst aus zick-zack-förmig geformten Drähten gebildet. Hierbei weist jeder Draht mindestens drei Schenkel auf, wobei der mittlere Schenkel mit den beiden angrenzenden Schenkeln jeweils einen spitzen Winkel einschließt.

Das aus mehreren versetzt zueinander angeordneten Drähten gebildete Stützgerüst ist in den Mantel aus Memory-Elastomer eingebettet. Hierbei verhindert der Mantel, daß das Stützgerüst von Gewebezellen durchwachsen wird.

Ein solcher Platzhalter weist eine umfangsseitig gleichmäßige Rückstellkraft auf. Er ist unkompliziert in seinem Aufbau und läßt sich dementsprechend einfach fertigen.

Durch die Merkmale des Anspruchs 6 kann ein Platzhalter bereitgestellt werden, der sich durch seine Längenstabilität auszeichnet.

Die Schenkel eines Drahts sind so ausgelegt, daß jeweils zwischen zwei Schenkeln ein Winkel eingeschlossen wird, der kleiner als 45° ist. Durch diese geometrische Auslegung verändert der Platzhalter auch dann seine Länge nicht, wenn er zusammengedrückt wird. Dies ist insbesondere bei der Planung einer Stenose von Bedeutung, da jetzt der Platzhalter exakt auf seine erforderliche Länge abgestimmt werden kann.

Wird auf den Platzhalter radial von außen Druck ausgeübt, so bewegt sich der zwischen zwei Schenkeln liegende Knickpunkt in einer drehförmigen Bewegung nach innen. Gleichzeitig bewegt sich jedoch das innen liegende Ende des Schenkels mit seinem Knickpunkt in einer gegenläufigen Bewegung nach außen. Dadurch heben sich die Bewegungen gegenseitig auf. Der Platzhalter bleibt in seiner gesamten Länge stabil.

Wird auf einen Platzhalter eine Kraft ausgeübt, die aus einer Richtung senkrecht zur Längsachse der Schenkel angreift, biegen sich die Drähte zur Mitte hin durch. Die jeweils benachbarten Drähte würden sich dann relativ voneinander entfernen. Dies wird jedoch durch die Einbettung der Drähte im elastischen Mantel verhindert.

Wenn eine Längenstabilität des Platzhalters nicht nötig oder nicht gewollt ist, kann der Platzhalter auch gemäß den Merkmalen des Anspruchs 7 so ausgebildet sein, daß der Winkel zwischen jeweils zwei Schenkeln größer oder gleich 45° und kleiner oder gleich 90° ist.

In bevorzugter Ausgestaltung der Erfindung greifen die einander benachbarten Drähte gemäß den Merkmalen des Anspruchs 8 verzahnungsartig ineinander. Damit werden die umfangsseitigen Rückstellkräfte des Platzhalters unterstützt.

Durch das Ineinandergreifen der jeweils benachbarten Drähte wird ein effektives Zusammenwirken des Stützgerüstes mit dem Mantel gewährleistet. Wird auf den Platzhalter Druck ausgeübt, entfernen sich die Schenkel relativ zueinander, wodurch im Mantel eine Zugspannung entsteht, die die Rückstellung bewerkstelligt. Mithin wird die Zugkraft im Mantel in eine Druckkraft transformiert. Diese steht der von außen einwirkenden Druckkraft entgegen und bewirkt so die entsprechende Reaktionskraft in den Drähten.

Um ein Verrutschen eines erfindungsgemäßen Platzhalters in der Körperröhre zu vermeiden, kann der Mantel über den Umfang verteilt mit Vorsprüngen versehen werden. Diese können regelmäßig oder unregelmäßig angeordnet sein. Denkbar ist es, die Vorsprünge durch Noppen, Haken oder Spitzen zu formen. Besonders vorteilhaft können die Vorsprünge aber in der Weise ausgebildet werden, daß die das Stützgerüst bildenden Drähte so gebogen oder tordiert werden, daß schuppenartige Vorsprünge aus dem Mantel herausgedrückt werden. Der Platzhalter hat dadurch eine riffelartige Oberflächenstruktur. Diese Konfiguration ist insgesamt schonender für die betroffenen Gefäßwände, da eine Abschnürung der die Gefäßwände umgebenden Blutgefäße vermieden wird.

Die Erfindung ist nachfolgend anhand von in den Zeichnungen dargestellten Ausführungsbeispielen näher erläutert. Es zeigen:
- Figur 1a: in einer perspektivischen Darstellung einen Platzhalter in einem zusammengezogenen Zustand;
- Figur 1b: den Platzhalter der Figur 1a in expandiertem Zustand;
- Figur 2a-c: weitere Ausführungsformen von Platzhaltern mit einem Mantel aus Memory-Elastomer;
- Figur 3: in der Seitenansicht einen Stützdraht mit drei Schenkeln und der Darstellung von Bewegungsvorgängen;
- Figur 4: einen abgewickelten Ausschnitt aus einem Platzhalter;
- Figur 5: einen Platzhalter mit einem geflochtenen Stützgerüst.

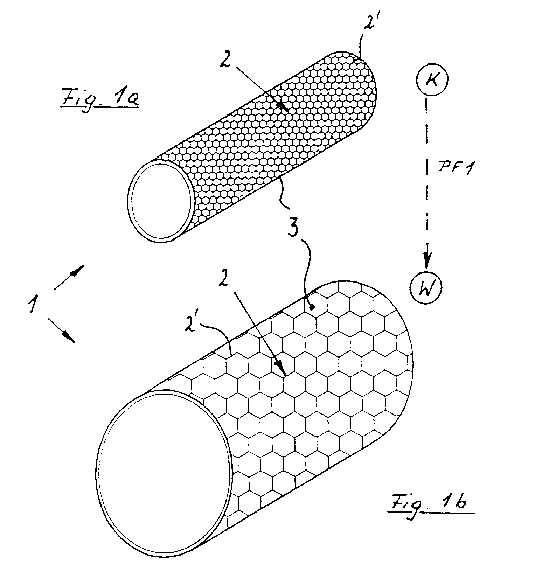

In den Figuren 1a und 1b ist ein Platzhalter 1 mit einem Stützgerüst 2 aus einer Formgedächtnislegierung und einem das Stützgerüst 2 einbettenden Mantel 3 aus einem Memory-Elastomer dargestellt. Das Stützgerüst 2 ist aus Drähten 2' gewoben.

Die Figur 1a stellt den Platzhalter 1 in einem kalten Zustand K dar. In dieser Phase ist der Mantel 3 aus dem Memory-Elastomer hart und klein. Das Stützgerüst 2 ist ebenfalls klein, aber verformbar, da das Memory-Metall plastisch weiche Eigenschaften besitzt.

So läßt sich der Platzhalter 1 in die von einer Stenose betroffene Körperröhre gut einführen.

Erst bei einer Temperatur, die geringfügig unter der Körpertemperatur liegt, kommt es zu einer Expansion des Platzhalters 1. Der Platzhalter 1 geht dann in den Zustand W über, der in der Figur 1b dargestellt ist. Der Übergang vom Zustand K zum Zustand W ist mit einem Pfeil PF1 für den Temperaturverlauf verdeutlicht.

Im Zustand W ist der Mantel 3 weich und flexibel; das Stützgerüst 2 hat sich entfaltet. Der Platzhalter 1 ist damit hart, aber elastisch. Er füllt das Lumen der betroffenen Körperröhre voll aus und ist in der Lage, dieses zu stützen.

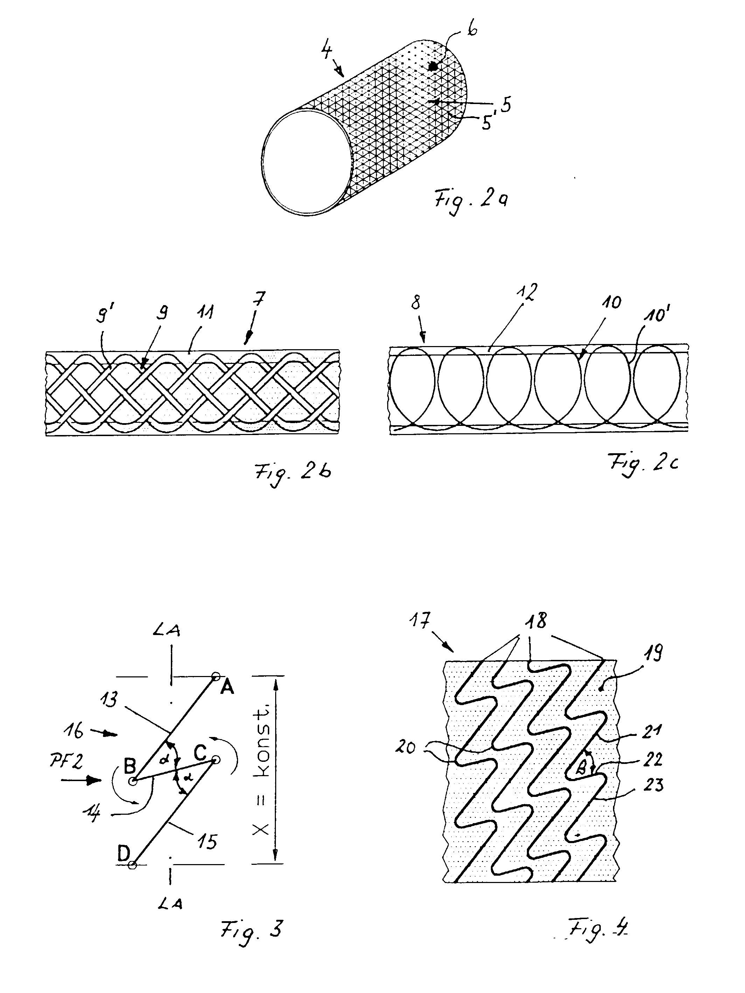

Die Figur 2a zeigt einen Platzhalter 4, dessen Stützgerüst 5 aus Nitinol-Drähten 5' eine netzartige Gewebestruktur aufweist. Das Stützgerüst 5 ist ebenfalls in einen Mantel 6 aus Memory-Elastomer eingebettet.

Vom Prinzip her den gleichen Aufbau weisen die Platzhalter 7 und 8 auf, von denen in den Figuren 2b und 2c jeweils ein Ausschnitt dargestellt ist. Sie zeichnen sich jeweils durch ein Stützgerüst 9, 10 aus Memory-Metall-Drähten 9' bzw. 10' aus, welches in einen Mantel 11, 12 aus Memory-Elastomer eingebettet ist.

In der Figur 3 sind drei Schenkel 13, 14, 15 eines Stützdrahtes 16 dargestellt. Der mittlere Schenkel 14 schließt mit den beiden angrenzenden Schenkeln 13 und 15 jeweils einen spitzen Winkel α ein. Die End- bzw. Knickpunkte zwischen den Schenkeln sind mit den Buchstaben A bis D gekennzeichnet.

Die Punkte A und D charakterisieren zwei auf den äußeren Enden des Platzhalters liegende Punkte und sollen als fixiert angesehen werden. Wird radial von außen entsprechend dem Pfeil PF2 auf den Knickpunkt B Druck ausgeübt, so bewegt sich der Knickpunkt B in einer drehförmigen Bewegung in der Bildebene nach unten und innen. Gleichzeitig bewegt sich der innenliegende Knickpunkt C in einer gegenläufigen Bewegung nach außen bzw. oben. Durch die sich überlagernden Bewegungen heben sich diese gegenseitig auf. Die vertikale Länge X des Stützdrahtes 16 bleibt folglich konstant. Die Endpunkte A und D bleiben ortsstabil.

Bei der Belastung des Drahts 16 mit einer Kraft, die senkrecht in die Bildebene gerichtet ist, wird sich der Draht 16 senkrecht zu seiner Längsachse LA durchbiegen.

Mithin bleibt ein Platzhalter, dessen Stützgerüst aus Stützdrähten entsprechend der Konfiguration von Stützdraht 16 aufgebaut ist, in seiner Gesamtlänge stabil, auch wenn er zusammengedrückt wird.

Einen Ausschnitt aus einem Platzhalter 17 zeigt Figur 4. Die Stützdrähte 18 aus Memory-Metall sind wiederum von einem Mantel 19 aus Memory-Elastomer umgeben.

Die einzelnen Drähte 18 greifen mit ihren gerundeten Längenabschnitten 20 verzahnungsartig ineinander. Zwischen den einzelnen Schenkeln 21, 22, 23 werden jeweils spitze Winkel β eingehalten. Dadurch ist der mittlere Schenkel 22 jeweils kürzer als die sich hieran anschließenden Schenkel 21 und 23.

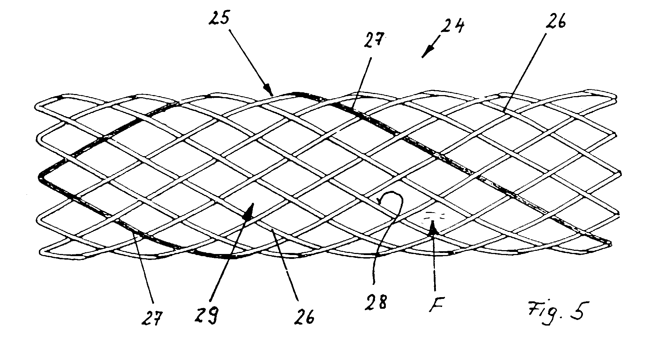

Aus der Figur 5 ist ein Platzhalter 24 ersichtlich, bei dem das Stützgerüst 25 aus einzelnen Fäden 26 und 27 flechttechnisch hergestellt ist. Die Fäden 26 bestehen aus einem temperaturempfindlichen Polyurethan, wohingegen die Fäden 27 aus Nitinol bestehen.

Bei einer Temperatur unterhalb von 25 °C sind die Fäden 26 hart und klein. Die Fäden 27 sind in diesem Temperaturbereich komprimiert, d.h. sie liegen in einem zusammengezogenen Zustand vor. Bei Erreichen der Grenztemperatur von 34 °C werden die Fäden 26 weich und elastisch und die Fäden 27 entfalten sich.

Bei einem Stent 24, der in ein Körpergefäß eingeführt ist, kann ein Feuchtigkeitsdurchgang von der äußeren Schleimhaut des Körpergefäßes in den inneren Bereich des Stents 24 stattfinden. Infolgedessen bildet sich an der inneren Wandung 28 ein Feuchtigkeitsfilm F auf.

Durch den rundgeflochtenen Aufbau zeichnet sich der Stent 24 weiterhin durch seine positiven Eigenschaften hinsichtlich des Biege- und Formverhaltens aus. Ein Einknicken der zylindrischen Konfiguration an Körperkrümmungen wird weitgehend vermieden.

Die Größe der Maschen 29 ist bei dem Stent 24 so gewählt, daß funktionsfähiges Gewebe hindurchwachsen kann.

## Patentansprüche

1. Platzhalter zum Anordnen in einer Körperröhre mit einem flexiblen Stützgerüst (25), welches aus mindestens einem Faden (26) besteht, der mit sich selber oder mit wenigstens einem weiteren Faden stabilisierend verflochten ist, **dadurch gekennzeichnet**, dass das Stützgerüst (25) aus einem Elastomer besteht, welches bei einer Temperatur kleiner 25°C hart ist und bei einer Temperatur größer 35°C weich ist, wobei mindestens ein weiterer Faden (27) aus einem Memory-Metall, insbesondere einer Nickel-Titan-Legierung, besteht.

2. Platzhalter (1, 4, 7, 8, 17) zum Anordnen in einer Körperröhre, der ein in einem hohlzylindrischen Mantel (3, 6, 11, 12, 19) aus Kunststoff eingebettetes flexibles Stützgerüst (2, 5, 9, 10) aufweist, **dadurch gekennzeichnet**, dass der Mantel (3, 6, 11, 12, 19) aus einem Memory-Elastomer, insbesondere auf Polyurethanbasis, besteht, welches bei einer Temperatur kleiner 25°C hart ist und bei einer Temperatur größer 35°C weich ist.

3. Platzhalter nach Anspruch 2, **dadurch gekennzeichnet**, dass das Stützgerüst (2, 5, 9, 10) aus metallischen Drähten (2', 5', 9', 10', 16, 18) gebildet ist.

4. Platzhalter nach Anspruch 3, **dadurch gekennzeichnet**, dass die Drähte (2', 5'. 9', 10', 16, 18) aus einem Memory-Metall, insbesondere einer Nickel-Titan-Legierung, gebildet ist.

5. Platzhalter nach einem der Ansprüche 2 bis 4, **dadurch gekennzeichnet**, daß das Stützgerüst (2, 5, 9, 10) aus mindestens zwei jeweils zick-zack-förmig geformten, sich parallel zur Längsachse (LA) des Mantels (3, 6, 11, 12, 19) erstreckenden und umfangsseitig des Mantels (3, 6, 11, 12, 19) zueinander versetzten Drähten gebildet ist, wobei jeder Draht (16, 18) mindestens drei Schenkel (13-15, 21-23) aufweist, von denen der mittlere Schenkel (14, 22) mit den beiden angrenzenden Schenkeln (13, 15, 21, 23) jeweils einen spitzen Winkel (α, β) einschließt.

6. Platzhalter nach Anspruch 5, **dadurch gekennzeichnet**, daß der spitze Winkel (α,β) kleiner als 45° ist.

7. Platzhalter nach Anspruch 5, **dadurch gekennzeichnet**, daß der spitze Winkel (α, β) größer oder gleich 45° und kleiner oder gleich 90° ist.

8. Platzhalter nach einem der Ansprüche 2 bis 7, **dadurch gekennzeichnet**, daß zwei umfangsseitig des Mantels (19) einander benachbarte Drähte (18) verzahnungsartig ineinandergreifen.

## Claims

1. A stent for disposing in a body tube and comprising a flexible supporting frame (25) made of at least one thread (26) which is stabilised by being woven with itself or with at least one additional thread, characterised in that the supporting frame (25) is of an elastomer which is hard at a temperature below 25°C and soft at a temperature above 35°C, at least one additional thread (27) being made of a memory metal, particularly a nickel-titanium alloy.

2. A stent (1, 4, 7, 8, 17) for disposing in a body tube and comprising a flexible supporting frame (2, 5, 9, 10) embedded in a hollow cylindrical plastic sleeve (3, 6, 11, 12, 19), characterised in that the sleeve (3, 6, 11, 12, 19) is made of a memory elastomer, particularly polyurethane-based, which is hard at a temperature below 25°C and soft at a temperature above 35°C.

3. A stent according to claim 2, characterised in that the supporting frame (2, 5, 9, 10) is made of metal wires (2', 5', 9', 10', 16, 18).

4. A stent according to claim 3, characterised in that the wires (2', 5', 9', 10', 16, 18) are made of a memory metal, particularly a nickel-titanium alloy.

5. A stent according to any of claims 2 to 4,
characterised in that the supporting frame (2, 5, 9, 10) is made up of at least two wires shaped in a zigzag, extending parallel to the longitudinal axis (LA) of the sleeve (3, 6, 11, 12, 19) and offset from one another peripherally of the sleeve (3, 6, 11, 12, 19), each wire (16, 18) being made up of at least three sections (13 - 15, 21 - 23), the middle section (14, 22) including an acute angle (α, β) with each of the two adjoining sections (13, 15, 21, 23).

6. A stent according to claim 5, characterised in that the acute angle (α, β) is less than 45°.

7. A stent according to claim 5, characterised in that the acute angle (α, β) is greater than or equal to 45° and less than or equal to 90°.

8. A stent according to any of claims 2 to 7,
characterised in that two wires (18) adjoining one another peripherally of the sleeve (19) interlock like teeth.

## Revendications

1. Extenseur ou dilatateur destiné à être mis en place dans un vaisseau corporel comportant une structure de soutien souple (25), qui consiste en au moins un fil (26) qui est tressé de façon stabilisée avec lui-même ou avec au moins un autre fil, caractérisé en ce que la structure de soutien (25) consiste en un élastomère qui a une température inférieure à 25°C dure et qui a une température supérieure à 35°C est mou, au moins au autre fil (27) en métal à mémoire, en particulier en un alliage nickel-titane.

2. Extenseur ou dilatateur (1,4,7,8,17) destiné à être mis en place dans un vaisseau corporel qui présente une structure de soutien souple (2,5,9,10) logée dans une enveloppe cylindrique creuse (3,6,11,12,19) en matière synthétique, caractérisé en ce que l'enveloppe (3,6,11,12,19) est réalisée en un élastomère à mémoire, en particulier à base de polyuréthane qui est dur à une température inférieure à 25°C et qui est mou à une température supérieure à 35°C.

3. Extenseur ou dilatateur selon la revendication 2, caractérisé en ce que la structure de soutien (2,5,9,10) est formée à partir de fils métalliques (2',5',9',10',16,18).

4. Extenseur ou dilatateur selon la revendication 3, caractérisé en ce que les fils (2' ,5' ,9' ,10' ,16, 18) sont formés à partir d'un métal à mémoire, en particulier d'un alliage nickel-titane.

5. Extenseur ou dilatateur selon l'une des revendications 2 à 4, caractérisé en ce que la structure de soutien (2,5,9,10) est formée par au moins deux fils en forme de zig-zag, s'étendant parallèlement à l'axe longitudinal (LA) de l'enveloppe (3,6,11,12,19) et décalée entre sur la périphérie de l'enveloppe (3,6,11,12,19), chaque fil (16,18) présentant au moins trois branches (13-15,21-23) dont la branche médiane (14,22) avec les deux branches limitrophes (13,15,21,23) inscrivent respectivement un angle aigu α,β.

6. Extenseur ou dilatateur selon la revendication 5, caractérisé en ce que l'angle aigu α,β est inférieur à 45°.

7. Extenseur ou dilatateur selon la revendication 5, caractérisé en ce que l'angle aigu α,β est supérieur ou égal à 45° et inférieur ou égale à 90°.

8. Extenseur ou dilatateur selon l'une des revendications 2 à 7, caractérisé en ce que deux fils (18) voisins sur la périphérie de l'enveloppe (19) s'imbriquent entre eux à la manière d'une denture.
